# EUROPEAN PATENT SPECIFICATION

(11) **EP 0 392 502 B1**
(45) Date of publication and mention of the grant of the patent: **07.12.1994**
(21) Application number: 90106943.5
(22) Date of filing: 11.04.1990
(51) Int. Cl.: G01N 1/22, G01N 33/00

(54) **Method and apparatus for measuring a parameter of a gas in isolation from gas pressure fluctuations**
Verfahren und Gerät zur Messung eines Gasparameters in Isolation von Gasdruckfluktuationen
Méthode et dispositif pour la mesure d'un paramètre d'un gaz isolé de fluctuations dans la tension de gaz

(30) Priority: 12.04.1989 US 336688
(43) Date of publication of application: 17.10.1990
(73) Proprietor: PURITAN-BENNETT CORPORATION, Carlsbad, California 92008 (US)
(72) Inventor: Goulding, Peter, San Diego, CA 92123 (US)
(74) Representative: Baillie, Iain Cameron

(56) References cited:
- EP-A- 0 006 256
- GB-A- 1 556 791
- US-A- 4 202 352

## Description

### BACKGROUND OF THE INVENTION

### Field of the Invention

The present invention relates generally to the measurement of parameters of gases and more particularly to a method and apparatus for measuring an unknown parameter of a gas in isolation from fluctuations in the pressure of the gas.

### Description of the Related Art

There are numerous instances in which an unknown parameter of a gas must be measured. For example, in the medical field it is often necessary to measure such parameters as oxygen and carbon dioxide concentrations in a gas mixture in either an inspiration or an expiration line of a patient ventilator. See e.g. US-A-4 202 352 which discloses an apparatus for measurement of expired gas in infants. Fluctuations in the pressure of the gas during the measurement process can affect the accuracy of the measurements or even render performance of the measurements impossible. Therefore, it is highly desirable to keep the pressure of the gas relatively constant while the measurements are being performed.

If the gas is not in motion, it is relatively easy to keep the pressure constant so as to carry out the necessary measurements. Even if the gas is in motion, if the pressure of the moving gas remains relatively constant, the concentration measurements can be performed satisfactorily. However, if the gas flow rate and pressure are not uniform, as is the case in an airway of a patient respirator, accurate parameter measurements become difficult or impossible.

Recent advances in medical diagnostic and therapeutic procedures have created a need for a way to continuously monitor parameters of the gases being inhaled or exhaled by a patient on a respirator. Various methods of measuring such parameters have been proposed, but none of them has adequately solved the problem of making accurate measurements under the conditions of fluctuating pressure and flow rate which are encountered in respirator airways. Therefore, there remains a need for a way to measure parameters of a gas such as a gas in a patient airway in isolation from fluctuations in the pressure and flow rate of the gas.

### SUMMARY OF THE INVENTION

According to one aspect of the invention, there is a system for measuring a parameter of a test gas, the system including a source of the test gas, a sensor in fluid communication with the source of the test gas operative to measure the parameter of the test gas, and pump means in fluid communication with the sensor to draw the test gas from the source of the test gas into the sensor, means for isolating the sensor from any fluctuations in the pressure of the test gas, an air capacitor upstream from the sensor in fluid communication therewith and operative to store a quantity of gas; a source of an alternate gas having an essentially constant pressure; fluid flow control means having a first fluid flow path connectable between the air capacitor and the source of the test gas, and a second fluid flow path connectable between the air capacitor and the source of alternate gas, the fluid flow control means being operative to open the first fluid flow path for the test gas into the air capacitor, to thereafter close said first flow path and to open the second fluid flow path between the air capacitor and the alternate source and to thereby isolate the air capacitor and the sensor from any fluctuations in the pressure of the test gas; indicator means in electrical communication with the sensor for providing an output signal representing the measure of the parameter of the test gas; characterized by a timing control means in electrical communication with the fluid flow control means and the indicator means for opening the first fluid flow path for a predetermined period of time until the air capacitor and the sensor are filled with the test gas, closing the first fluid flow path and opening the second fluid flow path, and for controlling the indicator means to commence providing the output signal a predetermined period of time after the second fluid flow path is opened when the pressure of the test gas is held constant, whereby the output signal is provided only when the pressure of the test gas in the sensor is held constant.

According to another aspect of the invention there is provided a method of measuring a parameter of a test gas in isolation from gas pressure fluctuations comprising the steps of opening a first fluid flow path for the test gas into an air capacitor; drawing the test gas through the air capacitor and into a sensor for measurement of the parameter of the test gas, thereby filling the air capacitor with the test gas; closing the first fluid flow path; opening a second fluid flow path between a constant pressure alternate gas source and the air capacitor and thereby isolating the sensor from any fluctuations in the pressure of the test gas; allowing pressure within the sensor to stabilize for a predetermined period of time after the second fluid flow path is closed before measuring the parameter of the test gas, and measuring the parameter of the test gas under constant pressure conditions before any substantial mixing of the alternate gas with the test gas stored in the air capacitor occurs.

The present invention provides a method and apparatus for measuring a parameter of a test gas in isolation from fluctuations in the pressure and flow rate of the gas by drawing the gas into an air capacitor and then admitting an alternative gas at a constant pressure to an inlet of the capacitor while the measurement is being performed.

Briefly and in general terms, apparatus according to the invention includes a sensor to measure the unknown parameter of the test gas, a pump downstream from the sensor for drawing the gas into the sensor, an air capacitor upstream from the sensor for storing a quantity of the gas, a constant pressure alternate gas source, and fluid flow control means.

According to the invention, the fluid flow control means opens a first fluid flow path for the test gas into the air capacitor for a sufficient time to enable the pump to draw the gas through the air capacitor into the sensor and thereby to fill the air capacitor with the test gas. Thereafter, the first flow path is closed and a second flow path between the air capacitor and the alternate gas source is opened, thereby isolating the air capacitor and the sensor from any fluctuations in the pressure of the test gas. The air capacitor prevents any substantial mixing of the alternate gas with the stored test gas for a sufficient time to enable the sensor to measure the unknown parameter of the test gas under constant pressure conditions. The pressure isolation provided by the air capacitor permits the test gas to flow continuously through the sensor during the measurement process while preventing pressure fluctuations from interfering with accuracy of the measurement.

In a preferred embodiment of the invention, the fluid flow control means comprises a valve, such as a solenoid valve, having a first inlet in fluid communication with a source of the test gas, a second inlet in fluid communication with the alternate gas source and an outlet in fluid communication with the air capacitor. The valve opens the first flow path by connecting the outlet to the first inlet and opens the second flow path by connecting the outlet to the second inlet.

The fluid flow control means preferably also includes timing control means such as a microprocessor or the like which selectively applies power to the solenoid valve to control the flow of gases into the air capacitor. The microprocessor also controls an output display or the like to ensure that the sensor output is provided only during the time that the pressure of the test gas in the sensor is being held constant. In a preferred embodiment, the test gas comprises an inspired gas in an airway of a patient respirator. The alternate gas source preferably comprises the ambient atmosphere.

The air capacitor preferably comprises an elongated chamber characterized by a length-to-diameter ratio much larger than unity; for example, a chamber having a length of about 75 centimeters and a diameter of about 0.5 centimeters has been found to give satisfactory results. The air capacitor can be fabricated, for example, by forming an elongated channel which establishes a fluid flow path between two cavities in a block of material, or can simply comprise a similar length of tubing having a similar diameter, and having appropriate adapters at each end.

According to the invention, a method of measuring an unknown parameter of a test gas in isolation from gas pressure fluctuations is provided that utilizes apparatus of the kind described above. The method comprises the steps set out in claim 11.

From the above, it may be seen that the present invention provides a system and method that enhances the accuracy and reliability of measurment of gas parameters in a patient's airway. These benefits are provided by a relatively simple and robust apparatus that does not interfere with the basic function of the respirator. Other aspects and advantages of the present invention will become apparent from the following detailed description, which taken in conjunction with the accompanying drawings, illustrate by way of example the principles of the invention.

### BRIEF DESCRIPTION OF THE DRAWINGS

FIGURE 1 is a fluid flow diagram of apparatus according to the invention for measuring a parameter of a gas in isolation from pressure fluctuations;
FIG. 2 is a perspective view of an air capacitor block located upstream of the sensor of the apparatus of Fig. 1;
FIG. 3 is a top plan schematic view of the air capacitor FIG. 2;
FIG. 4 is a top plan view of a pressure damping air capacitor block to be connected between the sensor and pump of FIG. 1; and
FIG. 5 is a sectional elevational view of an assembly of the pressure damping block and the air capacitor, taken along line 5-5 of FIG. 3, and line 5-5 of FIG. 4.

### DESCRIPTION OF THE PREFERRED EMBODIMENT

As shown in the drawings, which are included for purposes of illustration, the invention is embodied in a novel method and apparatus for measuring a parameter of a gas in isolation from fluctuations in the pressure of the gas. The monitoring of parameters such as oxygen and carbon dioxide concentrations in inspired or expired gases in a patient's airway has become of great importance with advances in medical technology, but the accuracy of the measurement of such parameters has been adversely affected by pressure fluctuations in the airway.

In accordance with the invention, a test gas is drawn into a sensor through an air capacitor which stores the test gas. Thereafter, a constant pressure alternate gas source is placed in fluid communication with the air capacitor, isolating the air capacitor and the sensor from any fluctuations in the pressure of the main body of the test gas while an unknown parameter of the test gas is being measured. The invention thus provides a simple and effective means for accurately measuring unknown parameters of the test gas notwithstanding fluctuations in the pressure of the main body of the test gas.

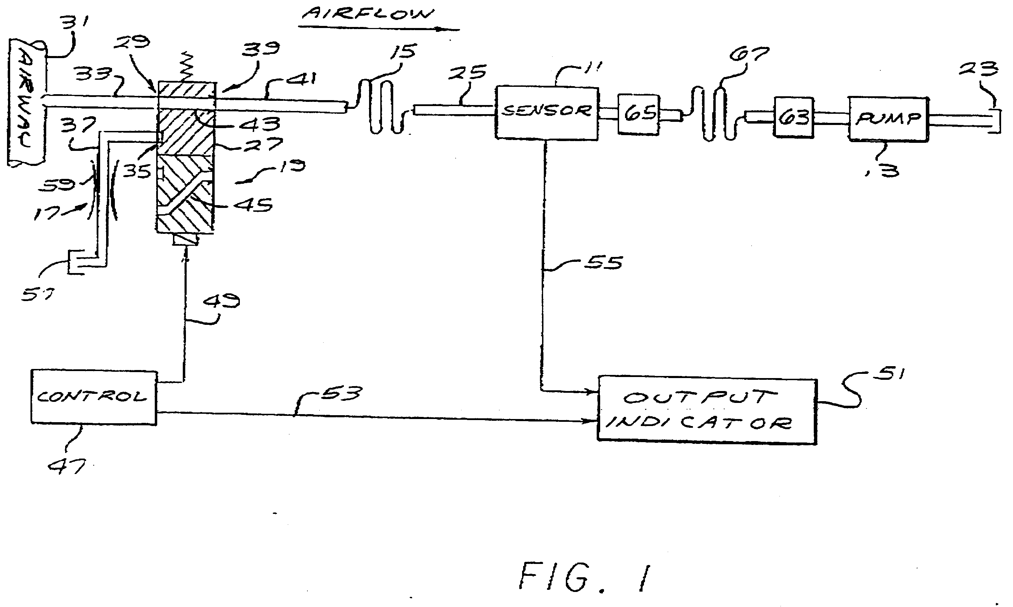

More particularly, as is shown schematically in FIGURE 1, an apparatus according to the invention for measuring an unknown parameter of a test gas in isolation from gas pressure fluctuations comprises a sensor 11 operative to measure the unknown parameter, a pump 13 downstream from the sensor 11, an air capacitor 15 upstream from the sensor 11, a constant pressure alternate gas source generally designated 17, and fluid flow control means generally designated 19.

The pump 13 is in fluid communication with the sensor 11 through fluid flow lines 75, 73 cavities 63 and 65 and channel 67. The pump 13 draws the test gas into the sensor 11 for measurement of the unknown parameter. The gas ultimately flows out of the sensor 11 into the pump 13 and is then discarded, for example, through an exhaust vent 23.

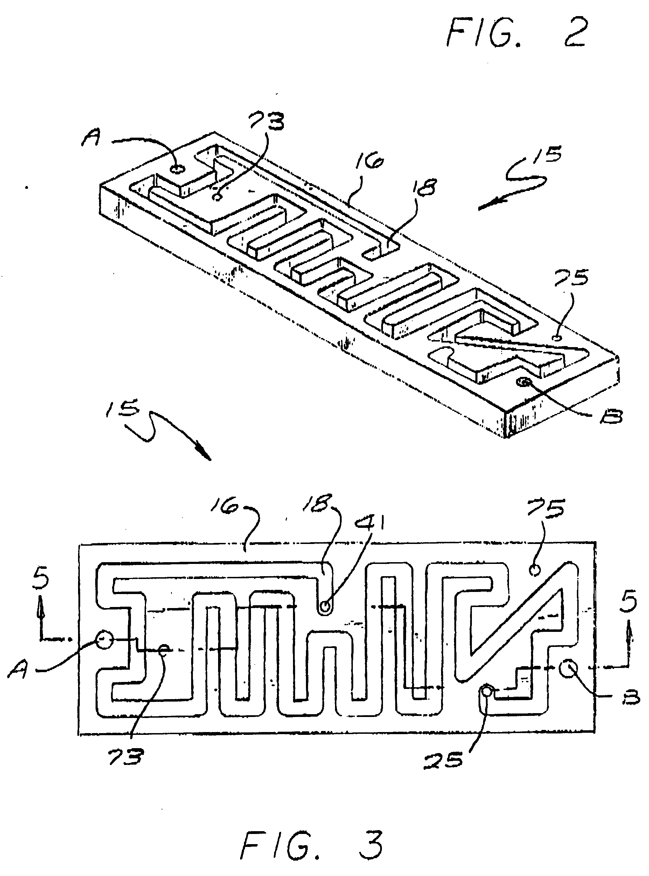

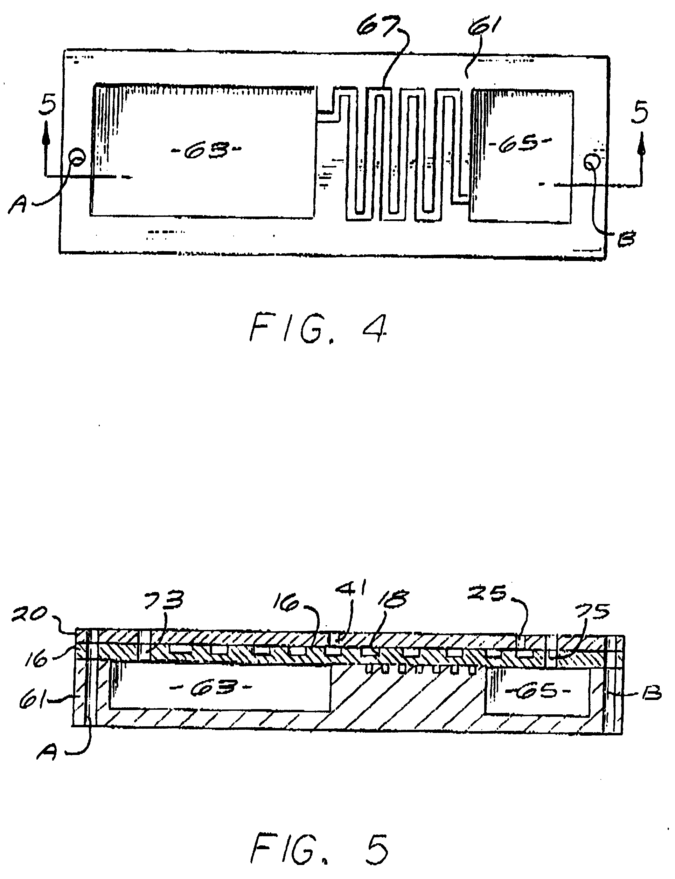

The air capacitor 15 in a currently preferred embodiment comprises a block 16 shown in Figures 2 and 3 having an enclosed channel 18 which is in fluid communication with the sensor 11 as shown by a fluid flow line 25 and stores a quantity of gas. The air capacitor channel 18 preferably comprises an elongated channel or chamber having a length-to-diameter ratio much larger than unity. For example, a channel having a length of about 75 centimeters and a diameter of about 0.5 centimeters has been found to be satisfactory. The air capacitor 15 is typically sealed by a cover 20 shown in Figure 5 to form the enclosed channel. Alternatively, the air capacitor may be formed by an appropriate length and diameter tubing which can be connected and function in a manner similar to the block form of the air capacitor.

The fluid flow control means 19 opens a first fluid flow path for the test gas into the air capacitor 15 for a sufficient time to enable the pump 13 to draw the test gas through the air capacitor 15 into the sensor 11 and thereby to fill the air capacitor 15 with said gas. Then the flow control means 19 closes the first fluid flow path and opens a second fluid flow path between the air capacitor 15 and the alternate gas source 17 and thereby isolates the sensor 11 from any fluctuations in the pressure of the test gas.

The air capacitor 15 prevents any substantial mixing of the alternate gas with the stored test gas for a sufficient time to enable the sensor 11 to measure the parameter of the test gas under constant pressure conditions without degrading the quality of the measurement. The pressure isolation provided by the air capacitor 15 permits the test gas to flow continuously through the sensor 11 during the measurement process while preventing pressure fluctuations from interfering with the accuracy of the measurement.

The fluid flow control means 19 preferably comprises a valve such as a solenoid valve 27 having a first inlet 29 in fluid communication with a source such as an airway 3 1 of the test gas as indicated by a fluid flow line 33, a second inlet 35 in fluid communication with the alternate gas source 17 as indicated by a fluid flow line 37, and an outlet 39 in fluid communication with the air capacitor 15 as indicated by a fluid flow line 41. The valve 27 opens the first fluid flow path by connecting the outlet 39 to the first inlet 29 as indicated by a first valve connection 43 and opens the second flow path by connecting the outlet 39 to the second inlet 35 as indicated by a second valve connection 45.

The fluid flow control means 19 preferably also includes timing control means 47 such as a microprocessor or the like which selectively applies power to the solenoid valve 27 as indicated by a control line 49 to control the flow of gases into the air capacitor 15. The timing control means 47 causes the valve 27 to keep the first fluid flow path open long enough to fill the sensor 11 and the air capacitor 15 with the test gas, then causes the valve 27 to close the first fluid flow path and to open the second fluid flow path to isolate the air capacitor 15 and the sensor 11 from any pressure fluctuations in the source of the test gas.

The timing control means 47 also controls an output indicator 51 or the like as indicated by a control line 53. The sensor 11 provides a parameter measurement signal to the output indicator 51 as indicated by a control line 55, and the timing control means 47 ensures that an output value is provided only during the time that the pressure of the test gas in the sensor 11 is being held constant. Preferably, the output value is delayed after the second fluid flow path has been opened just long enough for the pressure in the air capacitor 15 and the sensor 11 to stabilize.

In applications requiring frequent monitoring of an unknown parameter of the test gas, the timing control means 47 switches back and forth between the two fluid flow paths, the first path being kept open long enough to ensure that the air capacitor 15 and the sensor 11 are filled with the test gas and the second path being kept open long enough to ensure that pressures have stabilized before measurements are taken.

The alternate gas source 17 preferably comprises access to the ambient atmosphere, although other sources may be used. The second inlet 35 of the valve 27 is in fluid communication with the ambient atmosphere through the fluid flow line 37 and a vent 57. A flow constrictor 59 in the flow line 37 is preferably used to maintain constant pressure from the alternate gas source 17. In a preferred embodiment the test gas comprises inspired gas in the airway 31 of a patient respirator (not shown).

As shown in FIGS. 4 and 5, in an alternative embodiment, in order to damp out pressure fluctuations generated by the pump, the portion of the line 21 between the sensor and the pump may include a dual air capacitor block 61 which includes first and second cavities 63 and 65 and an elongated constrictor channel 67 establishing a resistor fluid flow path between the cavities 63 and 65, the channel 67 also having a length-to-diameter ratio much larger than unity.

The cavities 63 and 65 and the constrictor channel 67 are formed, for example, in an upper surface of the block 61. The block 61 may be covered by the bottom of the air capacitor block 16 and solvent bonded together to form a gas-tight seal with the adjacent cavities 63 and 65 and the channel 67 to constrain any gas flowing between the cavities 63 and 65 to flow through the channel 67. Fluid communication for a gas to enter and exit the cavities 63 and 65 is provided by means of orifices 73 and 75, respectively, extending through the block 16 and the cover 20. The block 16 and the cover 20 may be fabricated from various materials. Acrylic plastic or the like has been found to give satisfactory results.

A method according to the invention of measuring an unknown parameter of a test gas in isolation from gas pressure fluctuations by means of apparatus of the kind described above includes the steps as defined in claim 11.

From the foregoing it will be appreciated that the method and apparatus of the invention provide a way to accurately measure an unknown parameter of a gas which is subject to pressure fluctuations by storing the gas in an air capacitor and then isolating the air capacitor from the pressure fluctuations for a sufficient time to permit measurement of the unknown parameter. The invention is advantageously used, for example, to accomplish the accurate measurement of such parameters as oxygen and carbon dioxide concentration in inspired or expired gases in an airway of a patient respirator.

Although certain specific embodiments of the invention have been described and illustrated, the invention is not to be limited to the specific forms or arrangements of parts so described and illustrated, and various modifications and changes can be made without departing from the scope of the invention. Within the scope of the appended claims, therefore, the invention may be practiced otherwise than as specifically described and illustrated.

## Claims

1. A system for measuring a parameter of a test gas, the system including a source (31) of the test gas, a sensor (11) in fluid communication with the source (31) of the test gas operative to measure the parameter of the test gas, and pump means (13) in fluid communication with the sensor (11) to draw the test gas from the source (31) of the test gas into the sensor (11), means for isolating the sensor from any fluctuations in the pressure of the test gas,
an air capacitor (15) upstream from the sensor in fluid communication therewith and operative to store a quantity of gas;
a source of an alternate gas (17) having an essentially constant pressure;
fluid flow control means (19) having a first fluid flow path (29, 43) connectable between said air capacitor (15) and said source of the test gas, and a second fluid flow path (35, 45) connectable between said air capacitor (15) and said source of alternate gas (17), the fluid flow control means (19) being operative to open the first fluid flow path (29, 43) for the test gas into the air capacitor (15), to thereafter close said first flow path (29, 43) and to open the second fluid flow path (35,45) between the air capacitor (15) and the alternate source (17) and to thereby isolate the air capacitor (15) and the sensor (11) from any fluctuations in the pressure of the test gas;
indicator means (51) in electrical communication with said sensor (11) for providing an output signal representing the measure of the parameter of the test gas; characterized by:
a timing control means (47) in electrical communication with said fluid flow control means (19) and said indicator means (51) for opening said first fluid flow path (29, 43) for a predetermined period of time until said air capacitor (15) and said sensor (11) are filled with the test gas, closing said first fluid flow path (29, 43) and opening said second fluid flow path (35, 45), and for controlling said indicator means (51) to commence providing said output signal a predetermined period of time after said second fluid flow path (35, 45) is opened when said pressure of the test gas is held constant, whereby said output signal is provided only when the pressure of the test gas in the sensor is held constant.

2. The system according to Claim 1, wherein there is further provided means (61) for damping fluctuations in pressure in said test gas at said sensor due to said pump means (13) downstream from said sensor, said means for damping (61) including a first chamber (63) connected for fluid communication with said pump means (13), a second chamber (65) connected for fluid communication with said sensor (11), and an elongated constrictor channel (67) for establishing a resistor fluid flow path connected for fluid communication between said first and second chambers (63, 65).

3. The system according to Claim 1 or Claim 2, wherein the test gas is inspiration gas provided to a patient, and the fluid flow control means (19) comprises a valve (27) having a first inlet (29) in fluid communication with said source (31) of the test gas through a first flow path (43), a second inlet (35) in fluid communication with the alternate gas source (17) through a second flow path (45) and an outlet (39) in fluid communication with the air capacitor (15), the valve being operative to open the first flow path (43) by connecting the outlet (39) to the first inlet (29) and to open the second flow path (45) by connecting the outlet (39) to the second inlet (35).

4. The system according to any preceding claim, wherein the alternate gas source (17) comprises the ambient atmosphere.

5. The system according to any preceding claim, wherein the test gas is inspiration gas provided to a patient, and the source (31) of the test gas comprises an airway of a patient respirator.

6. The system according to any preceding claim, wherein the air capacitor (15) comprises an elongated chamber (18) characterized by a length-to-diameter ratio larger than unitY.

7. The system according to Claim 6, wherein the chamber (18) has a length of approximately 75 centimeters and a diameter of approximately 0.5 centimeters.

8. The system according to any preceding claim, wherein the air capacitor (15) comprises a block of material defining an elongated channel (18) establishing a fluid flow path having a length-to-diameter ratio larger than unity.

9. The system according to Claim 2 or according to any of Claims 3 to 8 when dependant on Claim 2, wherein said first and second chambers (63, 65) and said constrictor channel (67) of said damping means (61) are formed in a top surface of a first block member (61) covered by a bottom surface of a second block member (16), and said air capacitor (15) is formed in a top surface of said second block member (16) covered by a third block member (20).

10. The system according to Claim 9, wherein said second block member (16) includes a first pair of orifices (73, 75) therethrough in fluid communication with said first and second chambers (63, 65), said third block member includes a second pair of orifices (73, 75) therethrough, said first and second pairs of orifices being aligned to provide a first flow path (73) to said first chamber (63) and a second flow path (75) to said second chamber (65), and said third block member includes a third pair of orifices (73, 75) therethrough to provide a third and fourth flow path (73, 75) to said air capacitor (15).

11. A method of measuring a parameter of a test gas in isolation from gas pressure fluctuations characterized by the steps of opening a first fluid flow path (29, 43) for the test gas into an air capacitor (15); drawing the test gas through the air capacitor (15) and into a sensor (11) for measurement of the parameter of the test gas, thereby filling the air capacitor (15) with the test gas; closing the first fluid flow path (29, 43); opening a second fluid flow path (35, 45) between a constant pressure alternate gas source and the air capacitor (15) and thereby isolating the sensor (11) from any fluctuations in the pressure of the test gas; allowing pressure within said sensor (11) to stabilize for a predetermined period of time after said second fluid flow path (35, 45) is closed before measuring the parameter of the test gas, and measuring the parameter of the test gas under constant pressure conditions before any substantial mixing of the alternate gas with the test gas stored in the air capacitor (15) occurs.

12. The method according to Claim 11, wherein the step of opening a first fluid flow path (29, 43) comprises opening a fluid flow path (33, 29, 43) between an airway (31) of a patient respirator and the air capacitor (15).

## Patentansprüche

1. Anlage zur Messung eines Parameters eines Testgases, wobei die Anlage folgendes umfaßt: eine Quelle (31) für das Testgas, einen Sensor (11), der mit der Testgasquelle (31) in Fluidverbindung steht und den Parameter des Testgases messen kann, eine Pumpeneinrichtung (13), die mit dem Sensor (11) in Fluidverbindung steht, um das Testgas aus der Testgasquelle (31) in den Sensor (11) zu ziehen, und eine Einrichtung, die den Sensor gegen jegliche Schwankungen im Druck des Testgases abschirmt;
einen Luftkondensator (15), der stromauf von dem Sensor in Fluidverbindung mit diesem angeordnet ist und eine Gasmenge speichern kann;
eine alternative Gasquelle (17) mit einem im wesentlichen konstanten Druck;
eine Einrichtung (19) zur Steuerung der Fluidströmung mit einem ersten Fluidströmungsweg (29, 43), der zwischen dem Luftkondensator (15) und der Testgasquelle angeschlossen werden kann, und einem zweiten Fluidströmungsweg (35, 45), der zwischen dem Luftkondensator (15) und der alternativen Gasquelle (17) angeschlossen werden kann, wobei die Einrichtung (19) zur Steuerung der Fluidströmung den ersten Fluidströmungsweg (29, 43) öffnen kann, damit das Testgas in den Luftkondensator (15) strömt, um anschließend den ersten Strömungsweg (29, 43) zu schließen und den zweiten Fluidströmungsweg (35, 45) zwischen dem Luftkondensator (15) und der alternativen Gasquelle (17) zu öffnen und dadurch den Luftkondensator (15) und den Sensor (11) gegen jegliche Schwankungen im Druck des Testgases abzuschirmen;
eine Anzeigeeinrichtung (51), die mit dem Sensor (11) elektrisch verbunden ist, um ein Ausgangssignal abzusetzen, welches das Maß des Testgasparameters darstellt; gekennzeichnet durch:
eine zeitsteuereinrichtung (47), die mit der Einrichtung (19) zur Steuerung des Fluidstromes und mit der Anzeigeeinrichtung (51) elektrisch verbunden ist, um den ersten Fluidströmungsweg (29, 43) für einen vorbestimmten Zeitraum zu öffnen, bis der Luftkondensator (15) und der Sensor (11) mit dem Testgas gefüllt sind, um den ersten Fluidströmungsweg (29, 43) zu schließen und den zweiten Fluidströmungsweg (35, 45) zu öffnen, und um die Anzeigeeinrichtung (51) dahingehend zu steuern, daß sie anfängt, das Ausgangssignal für einen vorbestimmten Zeitraum nach dem Öffnen des zweiten Fluidströmungsweges (35, 45) abzusetzen, wenn der Druck des Testgases konstant gehalten wird, so daß das Ausgangssignal nur dann abgesetzt wird, wenn der Druck des Testgases in dem Sensor konstant gehalten wird.

2. Anlage nach Anspruch 1, bei der des weiteren eine Einrichtung (61) vorgesehen ist, die Schwankungen im Druck des Testgases an dem Sensor aufgrund der stromab von dem Sensor angeordneten Pumpeneinrichtung (13) dämpft, wobei die Dämpfungseinrichtung (61) eine erste Kammer (63) aufweist, die in Fluidverbindung mit der Pumpeneinrichtung (13) steht, eine zweite Kammer (65), die in Fluidverbindung mit dem Sensor (11) steht, und einen langgestreckten Verengungskanal (67), der einen Widerstandsströmungsweg bildet, der zur Herstellung einer Fluidverbindung zwischen der ersten und der zweiten Kammer (63, 65) angeschlossen ist.

3. Anlage nach Anspruch 1 oder Anspruch 2, bei der das Testgas ein einem Patienten zugeführtes Inspirationsgas ist und die Einrichtung (19) zur Steuerung des Fluidstromes ein Ventil (27) umfaßt, das einen ersten Einlaß (29) besitzt, der über den ersten Strömungsweg (43) mit der Testgasquelle (31) in Fluidverbindung steht, einen zweiten Einlaß (35), der über einen zweiten Strömungsweg (45) mit der alternativen Gasquelle (17) in Fluidverbindung steht, und einen Auslaß (39), der mit dem Luftkondensator (15) in Fluidverbindung steht, wobei das Ventil den ersten Strömungsweg (43) öffnen kann, indem es den Auslaß (39) mit dem ersten Einlaß (29) verbindet, und den zweiten Strömungsweg (45) öffnen kann, indem es den Auslaß (39) mit dem zweiten Einlaß (35) verbindet.

4. Anlage nach einem der vorhergehenden Ansprüche, bei der die alternative Gasquelle (17) die Umgebungsluft enthält.

5. Anlage nach einem der vorhergehenden Ansprüche, bei der das Testgas einem Patienten zugeführtes Inspirationsgas ist, und die Testgasquelle (31) einen Luftkanal eines Beatmungsgerätes für einen Patienten umfaßt.

6. Anlage nach einem der vorhergehenden Ansprüche, bei der der Luftkondensator (15) eine langgestreckte Kammer (18) umfaßt, die dadurch gekennzeichnet ist, daß das Verhältnis von Länge zu Durchmesser größer ist als Eins.

7. Anlage nach Anspruch 6, bei der die Kammer (18) eine Länge von ungefähr 75 Zentimetern und eine Durchmesser von ungefähr 0,5 Zentimetern besitzt.

8. Anlage nach einem der vorhergehenden Ansprüche, bei der der Luftkondensator (15) einen Materialblock umfaßt, der einen langgestreckten Kanal (18) begrenzt, der einen Fluidströmungsweg bildet, wo das Verhältnis von Länge zu Durchmesser größer ist als Eins.

9. Anlage nach Anspruch 2 oder nach einem der Ansprüche 3 bis 8, wenn diese von Anspruch 2 abhängen, bei der die erste und zweite Kammer (63, 65) und der Verengungskanal (67) der Dämpfungseinrichtung (61) in einer Oberseite eines ersten Blockelementes (61) ausgebildet sind, das von einer Unterseite eines zweiten Blockelementes (16) bedeckt wird, und bei der der Luftkondensator (15) in einer Oberseite des zweiten Blockelementes ausgebildet ist, das von einem dritten Blockelement (20) bedeckt wird.

10. Anlage nach Anspruch 9, bei der das zweite Blockelement (16) ein erstes Paar Öffnungen (73, 75) aufweist, die in Fluidverbindung mit der ersten und zweiten Kammer (63, 65) stehen, das dritte Blockelement ein zweites Paar Öffnungen (73, 75) aufweist, wobei das erste und das zweite Paar Öffnungen so ausgerichtet sind, daß ein erster Strömungsweg (73) zu der ersten Kammer (63) und ein zweiter Strömungsweg (75) zu der zweiten Kammer (65) gebildet wird, und bei der das dritte Blockelement ein drittes Paar Öffnungen (73, 75) aufweist, die einen dritten und vierten Strömungsweg (73, 75) zu dem Luftkondensator (15) bilden.

11. Verfahren zur Messung eines Testgasparameters isoliert gegen Gasdruckschwankungen, gekennzeichnet durch die folgenden Schritte: Öffnen eines ersten Fluidströmungsweges (29, 43), damit das Testgas in einen Luftkondensator (15) strömen kann; Einleiten des Testgases durch den Luftkondensator (15) und in einen Sensor (11), um den Parameter des Testgases zu messen, wodurch der Luftkondensator (15) mit dem Testgas gefüllt wird; Schließen des ersten Fluidströmungsweges (29, 43); Öffnen eines zweiten Fluidströmungsweges (35, 45) zwischen einer alternativen Gasquelle mit konstantem Druck und dem Luftkondensator (15) und auf diese Weise Isolieren des Sensors (11) gegen jegliche Schwankungen im Druck des Testgases; den Druck in dem Sensor (11) für einen vorbestimmten Zeitraum nach dem Schließen des zweiten Fluidströmungsweges (35, 45) sich stabilisieren lassen, bevor der Parameter des Testgases gemessen wird, und Messen des Parameters des Testgases bei konstantem Druck, bevor es zu einer starken Vermischung des alternativen Gases mit dem in dem Luftkondensator (15) gespeicherten Testgas kommt.

12. Verfahren nach Anspruch 11, bei dem der Schritt des Öffnens eines ersten Fluidströmungsweges (29, 43) das Öffnen eines Fluidströmungsweges (33, 29, 43) zwischen einem Luftkanal (31) eines Beatmungsgerätes für einen Patienten und dem Luftkondensator umfaßt.

## Revendications

1. Ensemble de mesure d'un paramètre d'un gaz éprouvé, l'ensemble comprenant une source (31) du gaz éprouvé, un capteur (11) communiquant avec la source (31) du gaz éprouvé et destiné à mesurer le paramètre du gaz éprouvé, et un dispositif (13) de pompage communiquant avec le capteur (11) afin qu'il aspire le gaz éprouvé de la source (31) du gaz éprouvé dans le capteur (11), un dispositif d'isolement du capteur des fluctuations de la pression du gaz éprouvé,
un accumulateur d'air (15) placé en amont du capteur et communiquant avec celui-ci et destiné à conserver une certaine quantité de gaz,
une source d'un autre gaz (17) ayant une pression pratiquement constante,
un dispositif (19) de réglage de débit de fluide, ayant un premier trajet (29, 43) de circulation de fluide et destiné à être connecter entre l'accumulateur d'air (15) et la source de gaz éprouvé, et un second trajet (35, 45) de circulation de fluide destiné à être raccordé entre l'accumulateur d'air (15) et la source d'un autre gaz (17), le dispositif (19) de réglage de débit de fluide étant destiné à ouvrir le premier trajet (29, 43) de circulation de fluide pour le gaz éprouvé vers l'accumulateur d'air (15), à fermer ensuite le premier trajet (29, 43) et à ouvrir le second trajet (35, 45) entre l'accumulateur d'air (15) et l'autre source (17) et à isoler ainsi l'accumulateur d'air (15) et le capteur (11) des fluctuations de la pression du gaz éprouvé, et
un dispositif indicateur (51) relié électriquement au capteur (11) et destiné à transmettre un signal de sortie représentant la mesure du paramètre du gaz éprouvé, caractérisé par :
un dispositif (47) de commande minutée relié électriquement au dispositif (19) de réglage de débit de fluide et au dispositif indicateur (51) afin qu'il ouvre le premier trajet (29, 43) pendant une période prédéterminée jusqu'à ce que l'accumulateur d'air (15) et le capteur (11) soient remplis du gaz éprouvé, qu'il ferme le premier trajet (29, 43) et ouvre le second trajet (35, 45), et qu'il commande le dispositif indicateur (51) de sorte qu'il commence à transmettre le signal de sortie une période prédéterminée après que le second trajet (35, 45) a été ouvert alors que la pression du gaz éprouvé est maintenue constante, si bien que le signal de sortie n'est transmis que lorsque la pression du gaz éprouvé dans le capteur est maintenue constante.

2. Ensemble selon la revendication 1, qui comporte en outre un dispositif (61) d'amortissement, au niveau du capteur, des fluctuations de pression du gaz éprouvé qui peuvent être dues au dispositif de pompage (13) placé en aval du capteur, le dispositif d'amortissement (61) comportant une première chambre (63) raccordée au dispositif de pompage (13) pour la circulation du fluide, une seconde chambre (65) raccordée afin qu'elle communique avec le capteur (11), et un canal allongé (67) de rétrécissement destiné à former un trajet de circulation de fluide présentant une résistance, raccordé afin qu'il assure la communication entre la première et la seconde chambre (63, 65).

3. Ensemble selon la revendication 1 ou 2, dans lequel le gaz éprouvé est un gaz aspiré transmis à un patient, et le dispositif (19) de réglage de débit de fluide comprend une soupape (27) ayant une première entrée (29) qui communique avec la source (31) du gaz éprouvé par un premier trajet (43) de circulation, et une seconde entrée (35) qui communique avec l'autre source (17) de gaz par un second trajet (45) de circulation et une sortie (39) communiquant avec l'accumulateur d'air (15), la soupape étant destinée à ouvrir le premier trajet (43) de circulation par raccordement de la sortie (39) à la première entrée (29) et à ouvrir le second trajet (45) de circulation par raccordement de la sortie (39) à la seconde entrée (35).

4. Ensemble selon l'une quelconque des revendications précédentes, dans lequel l'autre source de gaz (17) est l'atmosphère ambiante.

5. Ensemble selon l'une quelconque des revendications précédentes, dans lequel le gaz éprouvé est le gaz aspiré transmis à un patient, et la source (31) du gaz éprouvé comprend une voie de circulation d'air d'un appareil respiratoire d'un patient.

6. Ensemble selon l'une quelconque des revendications précédentes, dans lequel l'accumulateur d'air (15) comporte une chambre allongée (18) caractérisé par un rapport longueur-diamètre supérieur à l'unité.

7. Ensemble selon la revendication 6, dans lequel la chambre (18) a une longueur d'environ 75 cm et un diamètre d'environ 0,5 cm.

8. Ensemble selon l'une quelconque des revendications précédentes, dans lequel l'accumulateur d'air (15) comprend un bloc d'un matériau délimitant un canal allongé (18) établissant un trajet de circulation de fluide ayant un rapport longueur-diamètre supérieur à l'unité.

9. Ensemble selon la revendication 2 ou selon l'une des revendications 3 à 8 lorsqu'elle dépend de la revendication 2, dans lequel la première et la seconde chambre (63, 65) et le canal de rétrécissement (67) du dispositif d'amortissement (61) sont formés à une surface supérieure d'un premier organe (61) en forme de bloc couvert par une surface inférieure d'un second organe (16) en forme de bloc, et l'accumulateur d'air (15) est formé à la surface supérieure du second organe (16) en forme de bloc qui est couvert par un troisième organe (20) en forme de bloc.

10. Ensemble selon la revendication 9, dans lequel le second organe (16) en forme de bloc comprend une première paire d'orifices (73, 75) qui le traversent et qui communiquent avec la première et la seconde chambre (63, 65), le troisième organe en forme de bloc comporte une seconde paire d'orifices (73, 75) qui le traversent, la première et la seconde paire d'orifices étant alignées pour la formation d'un premier trajet (73) de circulation vers la première chambre (63) et d'un second trajet (75) de circulation vers la seconde chambre (65), et le troisième organe en forme de bloc comprend une troisième paire d'orifices (73, 75) qui le traversent et destinés à former un troisième et un quatrième trajet (73, 75) de circulation vers l'accumulateur d'air (15).

11. Procédé de mesure d'un paramètre d'un gaz éprouvé sous forme isolée des fluctuations de la pression du gaz, caractérisé par les étapes suivantes : l'ouverture d'un premier trajet (29, 43) de circulation de fluide du gaz éprouvé vers un accumulateur d'air (15), l'aspiration du gaz éprouvé, par l'intermédiaire de l'accumulateur d'air (15), dans un capteur (11) de mesure du paramètre du gaz éprouvé, avec remplissage de l'accumulateur d'air (15) du gaz éprouvé, la fermeture du premier trajet (29, 43) de circulation de fluide, l'ouverture d'un second trajet (35, 45) de circulation de fluide entre une source d'un autre gaz à pression constante et l'accumulateur d'air (15) avec isolement de cette manière du capteur (11) des fluctuations de la pression du gaz éprouvé, la stabilisation naturelle de la pression dans le capteur (11) pendant une période prédéterminée après la fermeture du second trajet (35, 45) de circulation de fluide avant la mesure du paramètre du gaz éprouvé, et la mesure du paramètre du gaz éprouvé dans des conditions de pression constante avant qu'un mélange notable de l'autre gaz ne se produise avec le gaz éprouvé conservé dans l'accumulateur d'air (15).

12. Procédé selon la revendication 11, dans lequel l'étape d'ouverture d'un premier trajet (29, 43) de circulation de fluide comprend l'ouverture d'un trajet (33, 29, 43) de circulation de fluide entre une voie de circulation (31) d'un appareil respiratoire d'un patient et l'accumulateur d'air (15).
